**Europäisches Patentamt**

**European Patent Office**

**Office européen des brevets**

(11) Veröffentlichungsnummer: **0 009 228**
B1

(12)

# EUROPÄISCHE PATENTSCHRIFT

(45) Veröffentlichungstag der Patentschrift:
14.10.81

(51) Int. Cl.³: **D 21 F 1/00, G 01 N 11/00**

(21) Anmeldenummer: **79103476.2**

(22) Anmeldetag: **17.09.79**

(54) **Vorrichtung zur wiederholten selbsttätigen Bestimmung der Entwässerungsfähigkeit einer Faserstoffsuspension.**

(30) Priorität: **18.09.78 DE 2840539**

(43) Veröffentlichungstag der Anmeldung:
**02.04.80 Patentblatt 80/7**

(45) Bekanntmachung des Hinweises auf die Patenterteilung:
**14.10.81 Patentblatt 81/41**

(84) Benannte Vertragsstaaten:
**AT BE CH DE FR GB IT NL SE**

(56) Entgegenhaltungen:
**US-A-3 198 006**

(73) Patentinhaber: **HAINDL PAPIER GMBH,
Georg-Haindl-Strasse 4, D-8900 Augsburg (DE)**

(72) Erfinder: **Eirenschmalz, Paul, Berg 8, D-8922 Peiting
(Birkland) (DE)**
Erfinder: **Schmid, Xaver, Füssener Strasse 38,
D-8922 Peiting (DE)**

(74) Vertreter: **Fuchs, Jürgen H., Dr.-Ing., Görtz, Dr. Fuchs, Dr.
Harders - Patentanwälte - Schneckenhofstrasse 27,
D-6000 Frankfurt am Main 70 (DE)**

Vorrichtung zur wiederholten selbsttätigen Bestimmung der Entwässerungsfähigkeit
einer Faserstoffsuspension

### Technisches Gebiet

Die Erfindung betrifft eine Vorrichtung zur wiederholten, selbsttätigen Bestimmung der Entwässerungsfähigkeit einer Faserstoffsuspension mit einer Einrichtung zum Zuführen der Faserstoffsuspension, einem an seiner einen Seite durch einen Siebboden verschlossenen und an seinem entgegengesetzten Einfüllende offenen, um eine horizontale Achse schwenkbaren Entwässerungsgefäß, welches mittels eines Drehantriebes mit seinem Einfüllende nach oben gerichtet unter die Faserstoffzuführeinrichtung und mit seinem Siebboden nach unten weisend über eine Filtratauffangeinrichtung einer Meßeinrichtung zum Auffangen und Messen einer durch den Siebboden ablaufenden Filtratmenge und ferner mit seinem Siebboden im wesentlichen nach oben gerichtet unter eine Spüleinrichtung bewegbar ist.

In der Fachwelt, vornehmlich im Bereich der Papierfabrikation seit langem bekannt und zur Bestimmung der Entwässerungsfähigkeit von Faserstoffsuspensionen im Labor gebräuchlich sind im deutschsprachigen Raum das sogenannte Schopper-Riegler-Gerät und im angelsächsischen Raum der Canadian-Freeness-Tester. Seit langem ist jedoch bereits das Ziel verfolgt worden, die Bestimmung der Entwässerungsfähigkeit selbsttätig intermittierend oder vollkontinuierlich unmittelbar in der Faserstoffaufbereitung beispielsweise einer Papiermaschine vornehmen zu können, um aus den Meßwerten direkte Rückschlüsse auf das Verhalten der Faserstoffsuspension auf der Papiermaschine ziehen und ohne wesentliche Zeitverzögerung entsprechende Regeleingriffe an der Papiermaschine vornehmen zu können.

Solche, im Produktionskreislauf anzuordnende Meßgeräte müssen vor allem zwei wichtigen Anforderungen genügen. Einmal müssen die von ihnen erhaltenen Meßwerte in einer reproduzierbaren Beziehung zum tatsächlichen Entwässerungsverhalten des Faserstoffes auf dem Sieb der Papiermaschine oder aber zum Regeldruck des Trockendampfes in der Papiermaschine stehen. Die zweite, ebenso wichtige Forderung liegt darin, daß die Geräte möglichst wartungsfrei und störungssicher arbeiten müssen, wenn man erreichen will, daß sich das Bedienungspersonal der Maschine auf die laufend vom Meßgerät gelieferten Werte verlassen können soll.

### Stand der Technik

Es hat einerseits nicht an Versuchen gefehlt, die im wesentlichen standardisierten Laborprüfgeräte für den vorgesehenen Zweck in ihrem Ablauf zu automatisieren, andererseits ist aber auch bereits eine Vielzahl von neu konzipierten Meßgeräten vorgeschlagen worden, die dem angegebenen Einsatzzweck dienen sollen.

Die Arbeitsweisen dieser Geräte unterscheiden sich darin, daß die Stoffprobe entweder unter dem sich ändernden Eigendruck ihrer eigenen Flüssigkeitssäule, unter dem Eigendruck einer konstanten Flüssigkeitssäule, unter hydrostatischem Überdruck oder unter Vakuum an der Filtratablaufseite des Siebes entwässert und daß entweder die Zeit, in der eine gewisse Filtratmenge anfällt, die Filtratmenge, die in einer gewissen Zeit anfällt, oder der sich an der Filtratablaufseite des Siebes einstellende Unterdruck bestimmt wird, wobei der eigentlichen Meßzeit eine konstante Vorentwässerungszeit oder eine Vorentwässerungszeit, in der eine bestimmte Filtratmenge anfällt, vorgeschaltet sein kann.

Die Meßvorrichtungen sind entweder so ausgebildet, daß auf einer stationären Siebfläche intermittierend eine Probe entwässert und dann das Sieb wieder gereinigt wird, oder aber auf einer sich fortbewegenden Siebfläche in hintereinander angeordneten Zonen kontinuierlich entwässert und wieder gereinigt wird. Bei beiden Vorrichtungsarten ist im wesentlichen zwischen zwei Typen zu unterscheiden, nämlich dem einen, bei dem das Entwässern des Stoffes von oben nach unten durch das Sieb und die Reinigung des Siebes von unten erfolgt, und dem anderen, bei dem die Faserstoffsuspensionsprobe im wesentlichen von unten gegen das Sieb gedrückt wird und das Ausspritzen des Siebes dann von oben nach unten erfolgt. Bei Vorrichtungen der ersteren Art bietet die Reinigung des Siebes im allgemeinen Schwierigkeiten, und es sind erhebliche Wassermengen erforderlich, um den auf dem Siebgewebe ausgebildeten Faserstoffkuchen entgegen der Schwerkraft aus der Meßzone fortzuspülen. Bei Geräten der letzteren Art erfordert die Einhaltung konstanter Entwässerungsbedingungen im allgemeinen einen verhältnismäßig hohen konstruktiven Aufwand. Bei kontinuierlichen Prüfgeräten, die sich eines um eine horizontale Achse rotierenden Rundsiebes bedienen, ist es schließlich möglich, das Entwässern wie auch das Spülen jeweils in der günstigsten Siebstellung vorzunehmen, jedoch erfordern solche Geräte ebenfalls einen verhältnismäßig hohen konstruktiven Aufwand.

Allgemein kann gesagt werden, daß Geräte, die eine verhältnismäßig gute Meßwertkorrelation liefern, entsprechend aufwendiger im Aufbau und daher auch störanfälliger sind. Die meisten bisher bekannt gewordenen Geräte sind daher entweder in ihrer Wirkungsweise nicht zufriedenstellend oder aber erfordern einen unvertretbar hohen Aufwand.

Aus US-A-3 198 006 ist ein intermittierend kontinuierlich arbeitendes Meßgerät bekannt, welches nach dem Meßverfahren des für Labormessungen vorgesehenen Canadian-Standard-Freeness-Testers arbeitet. Dieses Gerät ist dadurch automatisiert, daß zwei Entwässerungsgefäße vorgesehen sind, die an den Enden einer um eine vertikale Welle drehbaren Stange befestigt sind und sich somit karusselartig auf einer horizontalen Kreisbahn bewegen. Die Gefäße sind ihrerseits in einer vertikalen Ebene drehbar, so daß sie an bestimmten Stellen der Kreisbahn zum Befüllen und zum Ausführen des Meßvorgangs mit ihrer Öffnung nach oben und an anderer Stelle der Kreisbahn zum Freispülen der Siebe mit ihrer Öffnung nach unten gehalten werden können.

Dieses bekannte Gerät ist für Messungen an stark verdünnten Suspensionen entsprechend dem kanadischen Standardverfahren vorgesehen und für Messungen an Stoffen mit Büttenstoffdichte nicht geeignet. Beim Befüllen wird der Siebboden dadurch geschlossen gehalten, daß das Gefäß auf einer abdichtenden glatten Unterlage entlanggleitet, die dann für die Entwässerungsstrecke unterbrochen ist. Mit diesem Gerät sind nach dem Befüllen keine Vorentwässerungszeiten möglich, wie sie sich bei Messungen bei Büttenstoffdichte als erforderlich erwiesen haben. Dem gesamten Aufbau nach ist das bekannte Gerät für einen wartungsfreien Einsatz unter unmittelbaren Betriebsbedingungen nicht geeignet.

### Darstellung der Erfindung

Es ist die Aufgabe der vorliegenden Erfindung, eine einfache Meßvorrichtung der eingangs bezeichneten Art zu schaffen, welche Meßwerte liefert, die mit dem tatsächlichen Entwässerungsverhalten auf der Papiermaschine und mit dem zum Trocknen erforderlichen Dampfdruck in einer reproduzierbaren Beziehung stehen, mit der es möglich ist, Entwässerungsmessungen insbesondere auch bei üblichen, betrieblich anfallenden Büttenstoffdichten durchzuführen und die in einem solchen Maße wartungsfrei, störungsfrei und frei von Fremdeinflüssen arbeitet, daß die erhaltenen Meßwerte eine verläßliche Steuergröße für unmittelbare korrigierende Eingriffe im Produktionsablauf liefern.

Diese Aufgabe wird durch eine Vorrichtung der eingangs bezeichneten Art gelöst, welche erfindungsgemäß dadurch gekennzeichnet ist, daß das Entwässerungsgefäß als ein Entwässerungsrohr ausgebildet ist, dessen horizontale Schwenkachse ortsfest angeordnet ist, der Auslaufstutzen der Faserstoffzufuhreinrichtung und die Filtratauffangeinrichtung auf einer vertikalen Linie angeordnet sind, die durch den Drehpunkt des Entwässerungsgefäßes verläuft und die Austrittsöffnung der Spüleinrichtung seitlich des Auslaufstutzens in der Schwenkebene des Entwässerungsrohres angeordnet ist und der Drehantrieb direkt mit der ortsfesten Schwenkachse des Entwässerungsrohres verbunden ist.

Um eine Auffangeinrichtung für überschüssiges Filtrat, Spülstoff und Spülwasser zur Verfügung zu haben, ist das Entwässerungsrohr mit den unmittelbar zugehörigen Bauteilen vorteilhafterweise von einem Auffangbehälter umgeben, der an seinem unteren Ende mit einem trichterförmigen Ablauf versehen ist. Das hier anfallende Faserstoff-Wassergemisch kann in den Fabrikationskreislauf zurückgeführt werden. Um beim Füllen des Entwässerungsrohres ein starkes Stoffspritzen zu vermeiden, ist das Entwässerungsrohr zweckmäßigerweise mit einem seitlichen, schräg nach unten gerichteten Überlaufstutzen versehen, so daß der Stoffspiegel innerhalb des Rohres nur bis zur Oberkante dieses Stutzens ansteigen kann.

Ein Entwässerungsrohr zur Bestimmung der Entwässerungsfähigkeit von Faserstoffsuspensionen mit Stoffdichten zwischen 3,5 und 5,5% atro kann beispielsweise eine Länge von etwa 400—1000 mm und einen Durchmesser von 50—150 mm aufweisen.

Es hat sich gezeigt, daß die erfindungsgemäße Vorrichtung Ergebnisse liefert, die ausgezeichnet mit dem Stoffverhalten auf einer Papiermaschine korrelieren. Außerdem arbeitet die Vorrichtung praktisch wartungsfrei. Es sollten lediglich von Zeit zu Zeit Reinigungen vorgenommen werden, bei denen insbesondere auf die Entfernung von Verschleimungen im Bereich des Meßgefäßes für das Filtrat zu achten ist. Die Reinigung gestaltet sich äußerst einfach, da alle mit Stoff in Berührung befindlichen Teile des Gerätes sehr leicht zugänglich sind. Über den das Entwässerungsrohr umgebenden Auffangbehälter kann unmittelbar alles anfallende Reinigungswasser abgeleitet werden.

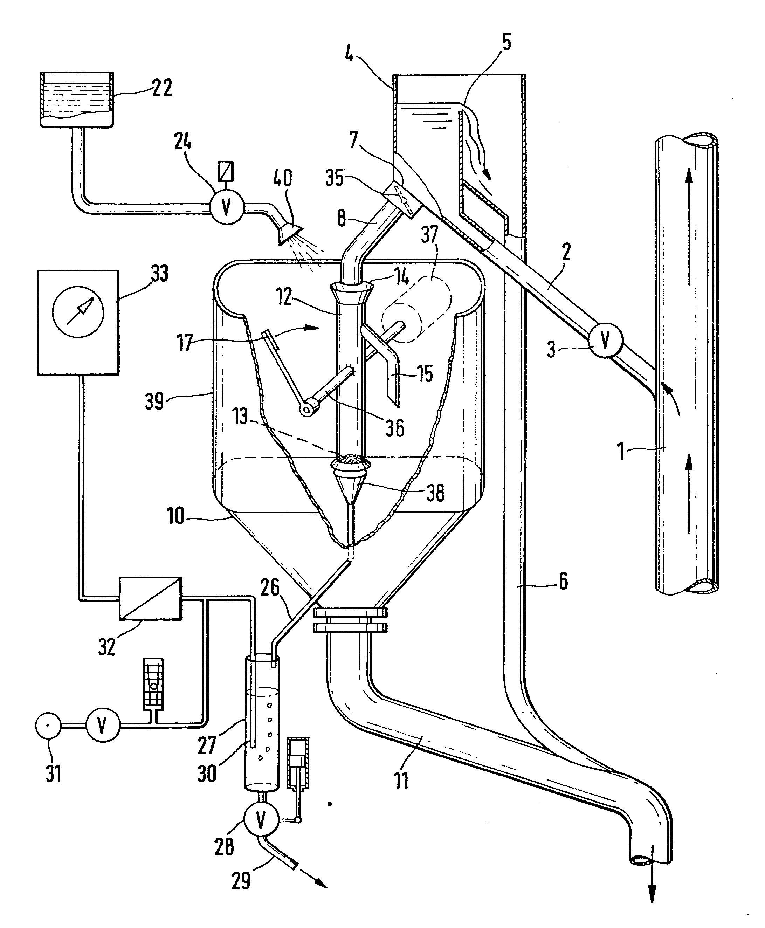

Im folgenden wird eine Ausführungsform der erfindungsgemäßen Vorrichtung unter Hinweis auf die beigefügte Zeichnung im einzelnen näher beschrieben.

### Kurzbeschreibung der Zeichnung

Die Figur stellt eine Vorrichtung zum Bestimmen der Entwässerungsfähigkeit von Faserstoffsuspensionen dar, bei der ein Entwässerungsrohr um eine stationäre, horizontale Achse in einer vertikalen Ebene schwenkbar ist.

### Beschreibung einer Ausführungsform der Erfindung

In der Figur ist ein Teil einer Stoffleitung 1 gezeigt, aus der über eine Abzweigleitung 2 ein kontinuierlicher Teilstrom der Faserstoffsuspension entnommen wird, der mittels eines Ventils 3, das zweckmäßigerweise als Blendenschieber ausgebildet ist, einstellbar ist.

Die Entnahmestelle hängt davon ab, an welcher Stelle des Produktionsprozesses die Entwässerungsfähigkeit der Faserstoffsuspension bestimmt werden soll. Wichtig ist für die Entnahme nur, daß es sich bei der Stoffleitung 1 um eine ständig durchströmte Leitung handelt, in der die Faserstoffsuspension unter Pumpendruck steht. Dabei kann der Faserstoff sogenannte Büttenstoffdichte besitzen, d. h. eine Stoffdichte zwischen etwa 2,5 und 5,5% atro (atro = absolut trocken). Die Stoffleitung kann eine direkte Förderleitung innerhalb des Produktionsprozesses sein, sie kann aber auch eine Umwälzleitung sein, über die der Faserstoff mittels einer Pumpe unten aus einer Bütte abgezogen und oben wieder in die Bütte zurückgeführt wird.

Der über die Abzweigleitung 2 abgetrennte kontinuierliche Teilstrom der Faserstoffsuspension wird von unten einem Überlaufkasten 4 mit konstantem Überlaufniveau zugeführt, welches durch eine Überlaufkante 5 gegeben ist. Der ständig anfallende Überlaufstoff wird über eine Rücklaufleitung 6 dem Prozeß wieder zugeführt.

In dem schrägen Boden 7 des die Faserstoffzuführeinrichtung für die Meßvorrichtung darstellenden Überlaufkastens 4 ist ein kurzer Auslaufstutzen 8 angeordnet. Die Lage dieses Auslaufstutzens 8 ist so gewählt, daß die in den Überlaufkasten 4 einströmende Faserstoffsuspension ständig am Eintritt des Auslaufstutzens vorbeiströmt, damit sich dort keine Ablagerungen bilden. Zwischen dem schrägen Boden 7 des Überlaufkastens 4 und dem Auslaufstutzen 8 ist eine Absperrklappe 35 vorgesehen. Um bei geschlossenem Zustand der Klappe eine Ansammlung von Faserstoff zu vermeiden, der sich während der Schließzeiten evtl. noch zusätzlich langsam entwässert und somit den Auslaufstutzen verstopfen kann, ist es zweckmäßig, die Absperrklappe dicht am Boden des Überlaufkastens anzuordnen. Andererseits soll aber auch der Auslaufstutzen 8 sehr kurz gehalten werden, damit bei geschlossener Klappe und damit unterbundenem Druckausgleich nicht Reste an Faserstoffsuspension unterhalb der Klappe im Stutzen hängen bleiben. Um dies zu vermeiden, ist es zweckmäßig, zusätzlich einen Luftschlitz in dem Auslaufstutzen vorzusehen. Wesentlich ist, daß die verwendete Absperrklappe dicht schließt, damit nicht während des Meßvorgangs, bei dem bei dieser Ausführungsform das Entwässerungsrohr 12 unterhalb des Auslaufstutzens 8 verbleibt, zusätzlicher Stoff oder Filtrat in das Entwässerungsrohr gelangen kann.

Unterhalb des Auslaufstutzens 8 ist die eigentliche Meßvorrichtung angeordnet, die innerhalb eines Auffangbehälters 39 angeordnet ist, dessen konisch zulaufendes Unterteil 10 mit einer Rücklaufleitung 11 versehen ist, die, wie im Beispiel dargestellt, mit der Rücklaufleitung 6 des Überlaufkastens 4 zusammengeführt ist, um alle aus der Meßvorrichtung anfallenden Stoffmengen dem Prozeß wieder zuführen zu können.

Das Kernstück der Vorrichtung zum Bestimmen der Entwässerungsfähigkeit ist das Entwässerungsrohr 12, das an seinem unteren Ende mit einem Siebboden 13 und an seinem oberen Ende mit einer trichterförmigen Erweiterung 14 zum leichteren Einfüllen des Faserstoffes versehen ist. Seitlich besitzt das Entwässerungsrohr 12 einen Überlaufstutzen 15, der das Niveau bestimmt, bis zu dem das Entwässerungsrohr für den Meßvorgang angefüllt werden soll. Ein solcher Überlaufstutzen hat sich als zweckmäßig erwiesen, da das Anfüllen des Rohres bis zu seiner Oberkante ein erhebliches Stoffspritzen verursachen würde.

Etwa in seinem mittleren Bereich ist das Entwässerungsrohr 12 an einer horizontalen, ortsfest drehbar in den Wänden des Behälters 39 gelagerten Achse 36 befestigt. An ihrem einen Ende trägt die Achse 36 ein an einem Hebelarm befestigtes Gegengewicht 17. Durch Drehen der Achse 36 ist das Entwässerungsrohr 12 in einer vertikalen Ebene schwenkbar, angetrieben durch einen am anderen Ende der Drehachse 36 angebrachten Antriebsmotor 37.

In der Ebene, in der das Entwässerungsrohr 12 um seine horizontale Achse 36 schwenkbar ist, ist oberhalb des Entwässerungsrohres der Auslaufstutzen 8 des Überlaufkastens 4 und seitlich davon eine Spüleinrichtung 22 angeordnet. Diese Spüleinrichtung besteht aus einer an eine Wasserleitung angeschlossenen, geeigneten Spüldüse 40, die mittels eines automatisch betätigbaren Wasserventils 24 einschaltbar ist.

Unterhalb des Entwässerungsrohres 12 ist ein Filtratauffangtrichter 38 angeordnet, der mit einem Ablaufrohr 26 versehen ist. Der Filtratauffangtrichter 38 und die Mündung des Auslaufstutzens 8 liegen auf einer vertikalen Linie, die durch den Drehpunkt des Entwässerungsrohres 12 verläuft. Das Ablaufrohr 26 mündet in ein Meßgefäß 27, das seinerseits mit einem durch ein automatisches Ventil 28 verschließbarem Ablaufrohr 29 versehen ist, welches ebenfalls in die Rücklaufleitung 11 des Auffanggefäßes 39 geführt sein kann. In das Meßgefäß 27 taucht ein Luftperlrohr 30 ein, welches von einer Steuerluftleitung 31 aus mit Meßluft beaufschlagbar ist. Die Meßluftleitung ist ferner mit einer einen Druckwandler enthaltenden Meß- und Auswerteinrichtung 32 verbunden, die zur Darstellung der Meßwerte beispielsweise mit einem Kreisblattschreiber 33 versehen ist.

Die Bestimmung der Entwässerungsfähigkeit einer Faserstoffprobe findet folgendermaßen statt:

Zu Beginn eines Meßzyklus befindet sich das von Faserstoff gesäuberte Entwässerungsrohr 12 in senkrechter Stellung mit seiner trichterförmigen Erweiterung 14 unter dem Auslaufstutzen 8. Zum Einfüllen einer Probe wird die Absperrklappe 35 im Auslaufstutzen 8 so lange geöffnet, bis der Füllstand des Stoffes im Entwässerungsrohr 12 die Höhe des Überlaufstutzens 15 mit Sicherheit erreicht hat, durch den überschüssiger Stoff seitlich abläuft. Sodann läßt man während einer bestimmten Vorentwässerungszeit, bei der das Ablaufventil 28 des Meßgefäßes 27 geöffnet ist, Filtrat weglaufen. Die sich dann anschließende Meßzeit beginnt mit dem Schließen des Ablaufventils 28 des Meßgefäßes 27 und endet mit dem Zeitpunkt, in dem begonnen wird, den Siebboden 13 des Entwässerungsrohres 12 über dem Filtratauffangtrichter 38 wegzuschwenken, um ihn zur Reinigung des Entwässerungsrohres an die Spüldüse 40 heranzuführen. Der sich im Meßgefäß 27 einstellende Filtratspiegel wird, wenn sich das Entwässerungsrohr 12 vom Auffangtrichter 38 wegbewegt hat, mit Hilfe des Perlrohrs 30 gemessen, und dieser Meßwert, der ein Maß für die Entwässerungsfähigkeit der untersuchten Stoffprobe ist, wird der Meßwertverarbeitungseinrichtung 32 zugeführt und in zeitlicher Folge mit den vorangegangenen Meßwerten auf dem Kreisblattschreiber 33 dargestellt. Nach erfolgter Niveaumessung wird das Meßgefäß 27 über das automatische Ableerventil 28 entleert.

Während das Entwässerungsrohr 12 mit seinem Siebboden 13 in Richtung auf die Spüldüse 40 geschwenkt wird, läuft die restliche Stoffsuspension weitgehend aus dem Entwässerungsrohr in den Behälter 39. Das Entwässerungsrohr 12 bewegt sich dann mit seinem Siebboden 13 nach oben unter der Spüleinrichtung 22 entlang, die durch automatische Betätigung des Ventils 24 in Funktion gesetzt wird, um den am Siebboden 13 etwa noch haftenden Faserstoffkuchen abzuspülen und das Entwässerungsrohr endgültig zu reinigen. Das Rohr wird dann wieder in seine aufrechte Stellung weitergedreht, und es kann ein neuer Meßvorgang beginnen.

Die für die Messung auszuwertende Entwässerungszeit wird einerseits durch die Öffnungs- bzw. Schließzeit des Ablaufventils 28 des Meßgefäßes 27 und zum anderen durch die Taktzeiten der Schwenkbewegung des Entwässerungsrohres 12 bestimmt, die beide durch Zeitrelais in weiten Grenzen einstellbar sind.

Die Mittel für die Programmsteuerung des beschriebenen Ablaufes sind weder in den Zeichnungen im einzelnen dargestellt, noch werden sie ausführlich beschrieben, da solche Programmabläufe nach Belieben mit bekannten Steuermitteln erreichbar sind.

Obwohl die Figur nur schematisch den Aufbau einer Vorrichtung zur selbsttätigen Bestimmung der Entwässerungsfähigkeit einer Faserstoffsuspension wiedergibt, so kann der Darstellung doch entnommen werden, daß sämtliche mit Faserstoffsuspension oder Wasser in Berührung kommenden Teile der Vorrichtung äußerst leicht zum Reinigen zugänglich sind, und daß die Vorrichtung insgesamt praktisch keine störanfälligen Bestandteile enthält.

**Patentansprüche**

1. Vorrichtung zur wiederholten, selbsttätigen Bestimmung der Entwässerungsfähigkeit einer Faserstoffsuspension mit einer Einrichtung (4, 8) zum Zuführen der Faserstoffsuspension, einem an seiner einen Seite durch einen Siebboden (13) verschlossenen und an seinem entgegengesetzten Einfüllende (14) offenen, um eine horizontale Achse (36) schwenkbaren Entwässerungsgefäß, welches mittels eines Drehantriebes (37) mit seinem Einfüllende (14) nach oben gerichtet unter die Faserstoffzuführeinrichtung (4, 8) und mit seinem Siebboden (13) nach unten weisend über eine Filtratauffangeinrichtung (38) einer Meßeinrichtung zum Auffangen und Messen einer durch den Siebboden (13) ablaufenden Filtratmenge und ferner mit seinem Siebboden (13) im wesentlichen nach oben gerichtet unter eine Spüleinrichtung (22, 24, 40) bewegbar ist, dadurch gekennzeichnet, daß das Entwässerungsgefäß als ein Entwässerungsrohr (12) ausgebildet ist, dessen horizontale Schwenkachse (36) ortsfest angeordnet ist, der Auslaufstutzen (8) der Faserstoffzufuhreinrichtung (4, 8) und die Filtratauffangeinrichtung (38) auf einer vertikalen Linie angeordnet sind, die durch den Drehpunkt des Entwässerungsgefäßes (12) verläuft und die Austrittsöffnung (40) der Spüleinrichtung (22, 24, 40) seitlich des Auslaufstutzens (8) in der Schwenkebene des Entwässerungsrohres (12) angeordnet ist und der Drehantrieb (37) direkt mit der ortsfesten Schwenkachse (36) des Entwässerungsrohres (12) verbunden ist.

2. Vorrichtung nach Anspruch 1, dadurch gekennzeichnet, daß der Drehpunkt des Entwässerungsrohres (12) etwa in dessen Mitte liegt.

3. Vorrichtung nach Anspruch 1, dadurch gekennzeichnet, daß das Entwässerungsrohr (12) unterhalb seiner Einfüllöffnung (14) mit einem im wesentlichen schräg abwärts gerichteten Überlaufstutzen (15) versehen ist.

4. Vorrichtung nach einem der Ansprüche 1 bis 3, dadurch gekennzeichnet, daß das Entwässerungsrohr (12) für Faserstoffsuspensionen mit etwa 3,5 bis 5,5% atro Stoffdichte eine Länge zwischen etwa 400 und 1000 mm und einen Durchmesser von vorzugsweise zwischen 50 und 150 mm aufweist.

**Claims**

1. Apparatus for the repeated automatic determination of the drainability of a fiber suspension, said apparatus comprising means (4, 8) for supplying the fiber suspension and a dewatering vessel pivotable about a horizontal axis (36) and having its one side closed by a screen bottom (13) and its opposite charging end (14) open, said dewatering vessel being positionable by means of a rotational drive (37) with its charging end (14) in an upwards pointing position under said means (4, 8) for supplying the fiber suspension and with its screen bottom (13) in a downwards pointing position over filtrate collecting means (38) of a measuring device for the collection and determination of a filtrate quantity drained through the screen bottom (13), and further with its screen bottom (13) in a generally upwards pointing position under a rinsing device (22, 24, 40), the apparatus being characterized in that the dewatering vessel has the configuration of a dewatering tube (12) the horizontal pivot axis (36) of which is stationary, that the outlet end (8) of said means (4, 8) for supplying the fiber suspension and said filtrate collecting means (38) are disposed on a vertical line traversing the pivot point of the dewatering tube (12), and the outlet port (40) of the rinsing device (22, 24, 40) is disposed laterally of said outlet end (8) within the plane of pivotal movement of the dewatering tube (12), and that the rotational drive (37) is directly connected to the stationary pivot axis (36) of the dewatering tube (12).

2. Apparatus according to claim 1, characterized in that the pivot point of the dewatering tube (12) is disposed approximately in the centre of the tube.

3. Apparatus according to claim 1, characterized in that the dewatering tube (12) is provided below its charging end (14) with a substantially downwardly inclined overflow stud (15).

4. Apparatus according to any one of claims 1 to 3, characterized in that for a fiber suspension with a consistency from 3,5 to 5,5% oven dry the dewatering tube (12) has a length between 400 and 1000 mm and a diameter of preferably between 50 and 150 mm.

**Revendications**

1. Dispositif permettant de déterminer, de manière répétitive et automatique, la capacité de déshydratation d'une suspension de matière fibreuse, et comprenant un mécanisme (4, 8) d'alimentation en suspension de matière fibreuse, ainsi qu'un récipient de déshydratation pouvant pivoter autour d'un axe horizontal (36), fermé à l'un de ses côtés par un fond perforé (13) et ouvert à son extrémité opposée de remplissage (14), un dispositif d'entraînement en rotation (37) pouvant : déplacer ledit récipient, dont l'extrémité de remplissage (14) est orientée vers le haut, sous ledit mécanisme (4, 8) d'alimentation en matière fibreuse; déplacer ce récipient, son fond perforé (13) étant orienté vers le bas, au-dessus du dispositif collecteur (38) de filtrat d'un dispositif de mesurage destiné à collecter et à mesurer une quantité de produit de filtration s'écoulant par ledit fond perforé (13); et déplacer ledit récipient sous un mécanisme de rinçage (22, 24, 40) lorsque le fond perforé (13) dudit récipient est orienté pour l'essentiel vers le haut, dispositif caractérisé par le fait que le récipient de déshydratation consiste en un tube de déshydratation (12) dont l'axe horizontal de pivotement (36) est stationnaire; par le fait que le raccord d'écoulement (8) du mécanisme d'alimentation (4, 8) et le dispositif collecteur (38) sont situés sur une ligne verticale passant par le point de rotation dudit tube de déshydratation (12); par lefait que l'orifice de sortie (40) du mécanisme de rinçage (22, 24, 40) occupe une position latérale par rapport audit raccord d'écoulement (8), dans le plan de pivotement dudit tube de déshydratation (12); et par le fait que le dispositif d'entraînement en rotation (37) est directement relié à l'axe stationnaire de pivotement (36) dudit tube de déshydratation (12).

2. Dispositif selon la revendication 1, caractérisé par le fait que le point de rotation du tube de déshydratation (12) so trouve sensiblement dans sa région médiane.

3. Dispositif selon la revendication 1, caractérisé par le fait que, au-dessous de son orifice de remplissage (14), le tube de déshydratation (12) comporte un manchon de trop-plein (15) essentiellement incliné vers le bas.

4. Dispositif selon l'une quelconque des revendications 1 à 3, caractérisé par le fait que le tube de déshydratation (12), destiné à traiter des suspensions de matières fibreuses d'une densité de matière entre environ 3,5% à 5,5% de siccité absolue, présente une longueur comprise entre environ 400 mm et 1000 mm et un diamètre compris, de préférence, entre 50 mm et 150 mm.